# EUROPÄISCHE PATENTSCHRIFT

(11) **EP 0 531 673 B1**
(45) Veröffentlichungstag und Bekanntmachung des Hinweises auf die Patenterteilung: **11.09.1996**
(21) Anmeldenummer: 92112353.5
(22) Anmeldetag: 20.07.1992
(51) Int. Cl.: C07D 201/02, C07D 207/26, C07D 211/76, C07D 223/10

(54) **Verfahren zur Herstellung von N-substituierten Lactamen**
Process for the preparation of N-substituted lactams
Procédé pour la préparation de lactames N-substitués

(30) Priorität: 01.08.1991 DE 4125457
(43) Veröffentlichungstag der Anmeldung: 17.03.1993
(73) Patentinhaber: BAYER AG, 51368 Leverkusen (DE)
(72) Erfinder: Botta, Artur, Dr., W-4150 Krefeld (DE); Buysch, Hans-Josef, Dr., W-4150 Krefeld (DE); Immel, Otto, Dr., W-4150 Krefeld (DE); Puppe, Lothar, Dr., W-5093 Burscheid (DE)

(56) Entgegenhaltungen:
- F. MOELLER 'HOUBEN-WEYL, BAND XI/1, STICKSTOFFVERBINDUNGEN II' 1957 , GEORG-THIEME VERLAG , STUTTGART
- CHEMISCHE TECHNOLOGIE April 1981, LEIPZIG Seiten 193 - 196

## Beschreibung

Die Erfindung betrifft ein Verfahren zur Herstellung von mit aliphatischen Resten N-substituierten Lactamen durch Umsetzung von entsprechend substituierten Lactam-N-carbonsäureestern oder Lactim-O-carbonsäureestern bei erhöhter Temperatur und gegebenenfalls in Gegenwart von Katalysatoren. Die erforderlichen Lactam-N-carbonsäureester bzw. Lactim-O-carbonsäureester können aus am Stickstoff unsubstituierten Lactamen und aliphatischen Carbonaten oder Pyrocarbonaten in situ hergestellt werden und ohne Zwischenisolierung sofort weiter zu den N-substituierten Lactamen umgewandelt werden.

Mit aliphatischen Gruppen am N-Atom substituierte Lactame, wie N-Methyl-pyrrolidon (NMP) oder N-Methylcaprolactam (NMC), sind wichtige technische aprotische Lösungsmittel, speziell für Extraktionen und Extraktivdestillationen; sie können auch als basische Verbindungen zur Entsäuerung von Erdgas oder Industriegasen eingesetzt werden (Chem. Techn. [Leipzig], 33 (4) (1981), 193-196).

Es ist bekannt, zur Herstellung von N-Alkyl-lactamen am Stickstoff unsubstituierte Lactame in der freien Form oder in der Alkalisalzform mit Alkylierungsmitteln, wie Alkylhalogeniden oder Dialkylsulfaten, umzusetzen (Houben-Weyl, Methoden der organischen Chemie, Stickstoffverbindungen II und III, Bd. XI/2, S. 569). Eine weitere Zusammenfassung des Standes der Technik zur Alkylierung von Caprolactam findet sich in der obigen Literaturstelle Chem. Techn. (loc. cit.). In dieser Publikation wird weiterhin über die Entwicklung eines technischen Verfahrens zur Herstellung von N-Methylcaprolactam berichtet, welches durch Umsetzung von Caprolactam mit 3 Mol Methanol als Alkylierungsmittel in der Gasphase an Tonerdekontakten gekennzeichnet ist; die Selektivität beträgt hierbei 90 %, der Umsatz beträgt jedoch nur 65 %. Bei diesem Verfahren entstehen gasförmige oder niedrigsiedende, übelriechende, aminartige Spaltprodukte, die zusammen mit dem entstehenden Prozeßwasser aufwendig entsorgt werden müssen (DD 226 563).

Es wurde nun ein Verfahren zur Herstellung von N-substituierten Lactamen der Formel 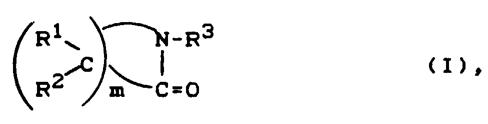 in der
- m: eine ganze Zahl von 2 bis 12 bedeutet,
- R¹ und R²: unabhängig voneinander Wasserstoff, geradkettiges oder verzweigtes C₁-C₆-Alkyl, geradkettiges oder verzweigtes C₃-C₆-Alkenyl, C₅-C₇-Cycloalkyl, Benzyl, Phenyl, Fluor, Chlor oder Brom darstellen, wobei an der durch den Index m bezeichneten Zahl von C-Ringgliedern höchstens 4 von Wasserstoff verschiedene Substituenten R¹ und R² vorhanden sind und wobei weiterhin eines oder zwei der in Klammern gesetzten C-Ringglieder durch -N(CH₃)- oder -N(C₂H₅)- ersetzt sein können, mehrere dieser C-Ringglieder durch Doppelbindungen verknüpft sein können oder 2 oder mehrere der in Klammern gesetzten C-Ringglieder Teile eines weiteren, aromatischen oder cycloaliphatischen Ringes sein können und die Reste R¹ und R² die ergänzenden Teile eines solchen Ringes darstellen, und
- R³: geradkettiges oder verzweigtes C₁-C₈-Alkyl, C₃-C₈-Alkenyl oder C₃-C₈-Alkinyl oder C₅-C₇-Cycloalkyl bedeutet,
gefunden, das dadurch gekennzeichnet ist, daß man Lactam-N-carbonsäureester oder Lactim-O-carbonsäureester der Formeln 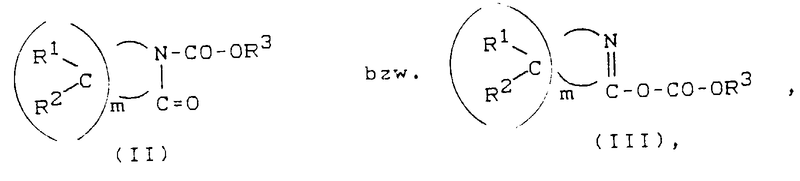 in denen m, R¹, R² und R³ die obige Bedeutung haben,
bei Temperaturen von 80-450°C in der Gas- oder der Flüssigphase und in Gegenwart oder Abwesenheit von Katalysatoren mit sauren und/oder basischen Zentren und in Gegenwart oder Abwesenheit eines inerten Lösungsmittels unter CO₂-Austritt spaltet, wobei im Falle des Einsatzes von Lactim-O-carbonsäureestern die intermediär entstehenden Lactim-ether unter den Reaktionsbedingungen in die N-substituierten Lactame umgelagert werden.

Geradkettiges oder verzweigtes C₁-C₈-Alkyl ist beispielsweise Methyl, Ethyl, Propyl, Isopropyl, Butyl, Isobutyl, die isomeren Pentyle, Hexyle oder Octyle. Bevorzugtes Alkyl hat 1-4 C-Atome und ist besonders bevorzugt Methyl oder Ethyl.

Geradkettiges oder verzweigtes C₃-C₆-Alkenyl ist beispielsweise Propenyl, Allyl, Butenyl, Pentenyl oder Hexenyl, Bevorzugtes Alkenyl ist Allyl und Butenyl.

Geradkettiges oder verzweigtes C₃-C₈-Alkinyl ist beispielsweise Propargyl, Butinyl, Pentinyl, Hexinyl oder Octinyl, bevorzugt Propargyl.

C₅-C₇-Cycloalkyl ist beispielsweise Cyclopentyl, Methylcyclopentyl, Cyclohexyl, Methyl-cyclohexyl oder Cycloheptyl, bevorzugt Cyclopentyl oder Cyclohexyl.

Der Index m bedeutet ganze Zahlen von 2 bis 12 und definiert damit Lactamgerüste mit 4-14 Ringgliedern. In bevorzugter Weise bedeutet der Index m die Zahlen 3, 4, 5 oder 12.

Lactam-N-carbonsäureester bzw. Lactim-O-carbonsäureester der Formeln (II) bzw. (III) lassen sich beispielsweise durch Umsetzung von Lactamen mit Chlorameisensäureestern in Gegenwart von tertiären Aminen oder durch Umsetzung von Lactimethern mit Chlorameisensäureestern herstellen (DE-OS 16 70 790; DE-OS 16 70 850; DE-OS 16 70 851; DE-PS 949 057).

In einer bevorzugten erfindungsgemäßen Variante lassen sich jedoch die genannten Lactam-N-carbonsäureester und die Lactim-O-carbonsäureester auch aus den am Stickstoff unsubstituierten Lactamen und aliphatischen Carbonaten oder Pyrocarbonaten in situ herstellen und, ohne daß sie isoliert werden müssen, unter CO₂-Abspaltung in die N-substituierten Lactame der Formel (I) weiter umwandeln.

Die Erfindung betrifft daher in dieser bevorzugten Variante ein Verfahren zur Herstellung von N-substituierten Lactamen der Formel (I), das dadurch gekennzeichnet ist, daß die einzusetzenden Lactam-N-carbonsäureester und Lactim-O-carbonsäureester durch Umsetzung von Lactamen der Formel 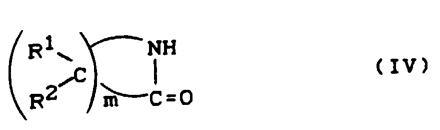 mit Carbonaten oder Pyrocarbonaten der Formel

R³O-CO-(O-CO)ₙ-OR³ (V),

worin
m, R¹, R² und R³ die oben angegebene Bedeutung haben, und
n 0 oder 1 bedeutet,
in einer Menge von 0,5-5 Mol, bevorzugt 1-3 Mol, besonders bevorzugt 1,2-1,7 Mol Carbonat oder Pyrocarbonat pro Mol Lactam bei Temperaturen von 80-450°C in der Gas- oder der Flüssigphase und in Gegenwart oder Abwesenheit von Katalysatoren mit sauren und/oder basischen Zentren und in Gegenwart oder Abwesenheit eines inerten Lösungsmittels in situ hergestellt werden und ohne Zwischenisolierung unter den gleichen Bedingungen und unter CO₂-Austritt in die N-substituierten Lactame umgewandelt werden.

Der Ablauf des erfindungsgemäßen Verfahrens unter Einbeziehung der bevorzugten Variante kann formelmäßig am Beispiel der Herstellung von NMC wie folgt dargestellt werden:

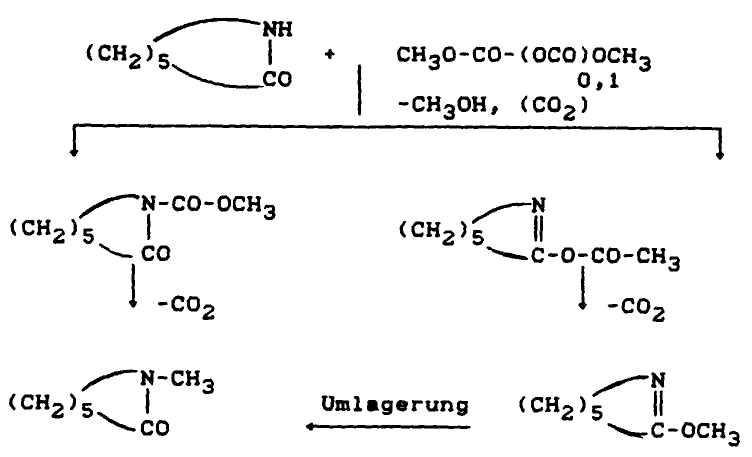

Aus dieser Darstellung ist erkennbar, daß das in der bevorzugten Verfahrensvariante eingesetzte Carbonat oder Pyrocarbonat kein Alkylierungsmittel sondern ein Acylierungsmittel ist und sich somit von Dimethylsulfat, Alkylhalogeniden oder Alkoholen unterscheidet. Unter den Bedingungen der bevorzugten Variante des erfindungsgemäßen Verfahrens sind jedoch die intermediär entstehenden Lactam-N- bzw. -O-carbonsäureester nicht beständig; sie werden vielmehr in situ unter CO₂-Austritt in die gewünschten N-substituierten Lactame umgewandelt. Die Umlagerung des Lactimethers in das N-substituierte Lactam ist bekannt.

In bevorzugter Weise werden erfindungsgemäß Lactamgerüste verstanden, in denen R¹ und R² durch R¹¹ und R¹² ersetzt werden, wobei
- R¹¹: Wasserstoff, geradkettiges oder verzweigtes C₁-C₄-Alkyl, Allyl, Butenyl, C₅-C₇-Cycloalkyl, Benzyl, Phenyl oder Chlor bedeutet und
- R¹²: für Wasserstoff, geradkettiges oder verzweigtes C₁-C₄-Alkyl oder Chlor steht.

In besonders bevorzugter Weise werden erfindungsgemäß Lactamgerüste verstanden, in denen R¹¹ und R¹² durch R²¹ und R²² ersetzt werden, wobei
- R²¹: Wasserstoff, Methyl, Ethyl, Benzyl oder Phenyl bedeutet und
- R²²: für Wasserstoff, Methyl oder Ethyl steht.

Die Reste R¹ und R² bzw. R¹¹ und R¹² bzw. R²¹ und R²² sind in den Lactamgerüsten, unabhängig von ihrer Ringgröße höchstens vierfach vorhanden, sofern sie von Wasserstoff verschiedene Substituenten darstellen. In bevorzugter Weise sind von Wasserstoff verschiedene Substituenten der genannten Art höchstens zweifach vorhanden. Die höchstens vierfach, bevorzugt höchstens zweifach vorhandenen genannten Substituenten können sowohl am gleichen C-Atom als auch an verschiedenen C-Atomen des Lactamgerüstes sitzen.

In weiterhin bevorzugter Weise werden N-substituierte Lactame hergestellt, in denen R³ durch R¹³ mit der Bedeutung geradkettiges oder verzweigtes C₁-C₄-Alkyl, Allyl oder Propargyl ersetzt ist. In besonders bevorzugter Weise werden N-substituierte Lactame hergestellt, in denen R¹³ durch R²³ mit der Bedeutung Methyl oder Ethyl ersetzt ist.

Weiterhin bevorzugte Lactame (IV) bzw. Lactam-Derivate (II) und (III) als Ausgangsmaterial für das erfindungsgemäße Verfahren sind solche, in denen der Formelteil 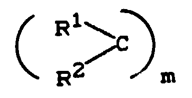 eine Kohlenstoffkette mit 2 oder 3 konjugierten C-C-Doppelbindungen, den 1,8-Naphthylen-Rest, den 2,2'-Biphenylen-Rest, den o-Phenylen-ethylen-Rest oder den o-Phenylenvinylen-Rest darstellt.

Lactame, die sich für das erfindungsgemäße Verfahren in seiner bevorzugten Variante eignen sind beispielsweise: β-Propiolactam, 2- und 3-Phenyl-β-propiolactam, 2- und 3-Methyl-β-propiolactam, 3-Ethyl-β-propiolactam, 3-Benzyl-β-propiolactam, 3,3-Dimethyl-β-propiolactam, γ-Butyrolactam, 4,4-Dimethyl-γ-butyrolactam, δ-Valerolactam, ε-Caprolactam, 2-Chlor-caprolactam, 2-, 3-, 4-, 5- und 6-Methyl-caprolactam, 2-, 3-, 4-, 5- und 6-Phenyl-caprolactam, substituiertes und nicht-substituiertes 7-Önantholactam, 8-Capryllactam, 12-Laurinlactam, Pyridon-2, Chinolon-2, Dihydrochinolon-2, Octahydrochinolon-2, Phthalimidin, Naphthostyril, Phenanthridinon-2, 2-Oxo-5,6-benz-tetrahydromzepin, 2-Oxo-6,7-benz-tetrahydroazepin, 7-Oxo-4-methyl-hexahydro-1,4-diazepin, 1H-2-Oxo-5-ethyl-1,5-diazacyclooctan, 1H-2-Oxo-1-aza-cyclooctatrien.

Die zuletzt (ab Pyridon-2) genannten Lactame können formelmäßig wie folgt dargestellt werden: 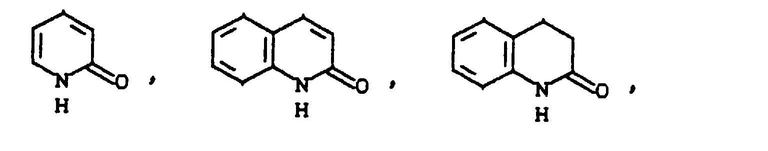 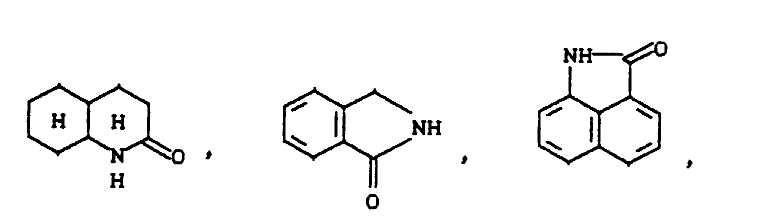 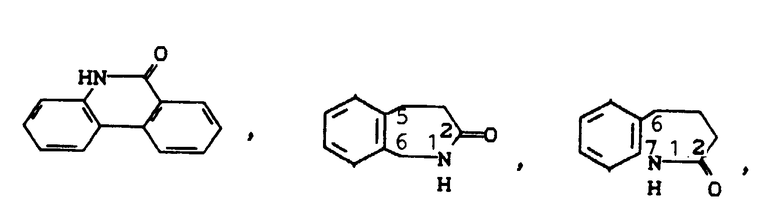 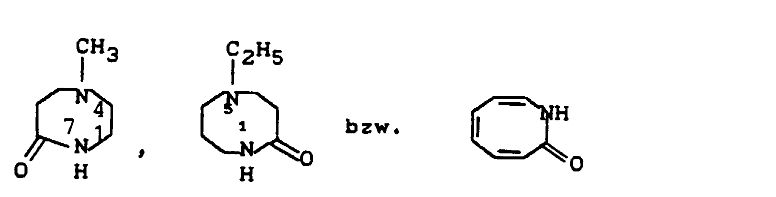 und zeigen Fälle auf, in denen in der Formel (I) in Klammern gesetzte C-Ringglieder durch -N(CH₃)- oder-N(C₂H₅)- ersetzt sind, durch Doppelbindungen verknüpft sind, oder in der genannten Art Teile von weiteren Ringen sind.

Für den Einsatz von Carbonaten in der bevorzugten Variante des erfindungsgemäßen Verfahrens eignen sich beispielsweise: Dimethylcarbonat, Diethylcarbonat, Dipropylcarbonat, Diisopropylcarbonat, Dibutylcarbonat, Diisobutylcarbonat, Di-sec.-butyl-carbonat, Dipentylcarbonat, Dihexylcarbonat, Dioctylcarbonat, Dicyclohexylcarbonat, Dioctylcarbonat, Dipentenylcarbonat, Dibenzylcarbonat sowie die entsprechenden Pyrocarbonate.

Das Carbonat oder Pyrocarbonat der Formel (V) wird in einer Menge von 0,5-5 Mol, bevorzugt 1-3 Mol, besonders bevorzugt 1,2-1,7 Mol pro Mol Lactam (IV) eingesetzt.

Das erfindungsgemäße Verfahren kann in Gegenwart oder in Abwesenheit eines inerten Lösungsmittels durchgeführt werden. Solche Lösungsmittel sind beispielsweise (Halogen)Kohlenwasserstoffe, wie Hexan, Cyclohexan, Petrolether, Ligroin, Benzol, Toluol, Xylol, Chlorbenzol, Dichlorbenzol, Naphthalin, Decalin oder Alkohole, wie Methanol, Ethanol, (Iso)propanol, (Iso)butanol, Ester, wie Ethyl-und Butylacetat, N-persubstituierte Amide, wie Dimethylformamid und -acetamid, N-Methyl-pyrrolidon (NMP) und -caprolactam (NMC) und andere, die sich unter den Bedingungen des erfindungsgemäßen Verfahrens inert verhalten. Lösungsmittel werden vor allem dann eingesetzt, wenn die umzusetzenden Ausgangsverbindungen höherschmelzend sind oder wenn beispielsweise in der Rieselphase oder der Flüssigphase gearbeitet werden soll.

Das erfindungsgemäße Verfahren kann weiterhin in Gegenwart oder in Abwesenheit von Katalysatoren mit sauren und/oder basischen Zentren durchgeführt werden. Die Gegenwart von solchen Katalysatoren erlaubt es in der Regel, die Reaktionstemperatur innerhalb des angegebenen Bereiches abzusenken und so das gesamte Reaktionsgemisch vor thermischer Überbeanspruchung zu schützen.

Katalysatoren mit sauren oder vorwiegend sauren Zentren sind dem Fachmann bekannt. Sie gehören verschiedenen Stoffklassen an, beispielsweise
den Tonmineralien, wie Kaolinite, Bentonite, Montmorillonite oder Attapulgite;
den Säuren oder freie Säuren oder Säuregruppen enthaltenden Trägern, wie Salzsäure, Salpetersäure, Schwefelsäure, Phosphorsäure, Oxalsäure, Essigsäure, Trifluoressigsäure, Benzolsulfonsäure oder Toluolsulfonsäure, Kationenaustauscherharze in der H⁺-Form oder Aktivkohle, SiO₂, Al₂O₃ oder Tonmineralien, die mit den genannten Säuren getränkt sind;
der Gruppe der überwiegend sauer reagierenden Metalloxide und Metallsulfide, wie Zinkoxid, Cadmiumoxid, Aluminiumoxid, Ceroxid, Thoriumoxid, Titanoxid, Zirkonoxid, Zinnoxid, Nioboxid, Tantaloxid, Bleioxid, Arsenoxid, Wismutoxid, Antimonoxid, Vanadinoxid, Chromoxid, Molybdänoxid, Wolframoxid, Cadmiumsulfid und Zinksulfid;
der Gruppe der Metallsalze, wie MgSO₄, CaSO₄, SrSO₄, BaSO₄, CuSO₄, ZnSO₄, CdSO₄, Al₂(SO₄)₃, FeSO₄, Fe₂(SO₄)₃, CoSO₄, NiSO₄, Cr₂(SO₄)₃, KHSO₄, K₂SO₄, (NH₄)₂SO₄, Zn(NO₃)₂, Ca(NO₃)₂, Bi(NO₃)₃, Fe(NO₃)₃, CaCO₃, BPO₄, AlPO₄, CrPO₄, Cu₃(PO₄)₂, Zn₃(PO₄)₂, Mg₃(PO₄)₂, Ti₃(PO₄)₄, Zr₃(PO₄)₄, Ni₃(PO₄)₂, AgCl, CuCl₂, CuCl, CaCl₂, AlCl₃, TiCl₃, SnCl₂, CaF₂, BaF₂, Al(ClO₄)₃, Mg(ClO₄)₂ und andere sowie Mischungen mehrerer der genannten Salze;
der Gruppe der gemischten Oxide, die auch in verschiedenen Mischungsverhältnissen mehrerer von ihnen eingesetzt werden können, wie SiO₂-Al₂O₃, SiO₂-TiO₂-SiO₂-SnO₂, SiO₂-ZrO₂, SiO₂-BeO, SiO₂-MgO, SiO₂-CaO, SiO₂-SrO, SiO₂-BaO, SiO₂-ZnO, SiO₂-Ga₂O₃, SiO₂-Y₂O₃, SiO₂-La₂O₃, SiO₂-MoO₃, SiO₂-WO₃, SiO₂-V₂O₅, SiO₂-ThO₂, Al₂O₃-MgO, Al₂O₃-ZnO, Al₂O₃-CdO, Al₂O₃-B₂O₃, Al₂O₃-ThO₂, Al₂O₃-TiO₂, Al₂O₃-ZrO₂, Al₂O₃-V₂O₅, Al₂O₃-MoO₃, Al₂O₃-WO₃, Al₂O₃-Cr₂O₃, Al₂O₃-Mn₂O₃, Al₂O₃-Fe₂O₃, Al₂O₃-Co₃O₄, Al₂O₃-NiO, TiO₂-CuO, TiO₂-MgO, TiO₂-ZnO, TiO₂-CdO, TiO₂-ZrO₂, TiO₂-SnO₂, TiO₂-Bi₂O₃, TiO₂-Sb₂O₅, TiO₂-V₂O₅, TiO₂-Cr₂O₃, TiO₂-MoO₃, TiO₂-WO₃, TiO₂-Fe₂O₃, TiO₂-Mn₂O₃-TiO₂-Co₃O₄, TiO₂-NiO, ZrO₂-CdO, ZnO-MgO, ZnO-Fe₂O₃, MoO₃-CoO-Al₂O₃, MoO₃-NiO-Al₂O₃, TiO₂-SiO₂-MgO, MoO₃-Al₂O₃-MgO, Heteropolysäuren und andere;
der Gruppe der sogenannten festen Supersäuren, wie SbF₅ oder TaF₅ und andere auf Trägern wie SiO₂, Al₂O₃, SiO₂-Al₂O₃, SiO₂-TiO₂, SiO₂-ZrO₂, TiO₂-ZrO₂, MoO₃, ThO₂-Cr₂O₃, Al₂O₃-WO₃ und anderen;
der Gruppe der Zeolithe, wie Zeolith X, Zeolith Y, Mordenit, Zeolith L, ZSM 5, [B]ZSM 5, [Fe]ZSM 5, [Ti]ZSM 5, [Ga]ZSM 5, [Cr]ZSM 5, ZSM 11, Zeolith EU1, ZSM 48, ZSM 12, ZSM 22, ZSM 23, Erionit, Offretit, Mazzit, Chabasit, PSH3, Zeolith β;
der mikroporigen kristallinen Zeolithanaloga, wie ALPOs, SAPOs und MeAPOs, und andere, die in der H-Form, aber auch in der mit Metallkationen ausgetauschten Form, wie dem Fachmann bekannt ist, weiterhin saure Zentren aufweisen, so insbesondere mit Mg, Ca, Zn, Cu, Sn, Seltenerd, aber auch mit K, Rb, Cs, Sr, Ba, in denen der basische Charakter zunimmt.

Katalysatoren mit vorwiegend basischen Habitus, die sich für die erfindungsgemäße Reaktion eignen, sind beispielsweise Metallhydroxide, -oxide, -alkoholate und -hydride der Alkali- oder Erdalkalimetallreihe, z.B. LiOH, NaOH, KOH , Sr(OH)₂, Ba(OH)₂, Na₂O, CaO, SrO, BaO, NaOCH₃, NaOC₂H₅, KOC₄H₉, NaH, Alkali- und Erdalkalicarbonate oder -hydrogencarbonate, wie Li₂CO₃, Na₂CO₃, NaHCO₃, K₂CO₃, KHCO₃, Cs₂CO₃, MgCO₃, CaCO₃, SrCO₃, BaCO₃.

Das erfindungsgemäße Verfahren kann sowohl in der Flüssigphase als auch in der Gasphase oder Rieselphase durchgeführt werden. Hierbei kann unter Normaldruck, erniedrigtem oder erhöhtem Druck gearbeitet werden. Das Arbeiten in der Flüssigphase, beispielsweise in einem Autoklaven eignet sich hierbei für diskontinuierliche Reaktionsansätze, während die Gasphase oder die Rieselphase vorwiegend für kontinuierliche Verfahren im technischen Maßstab geeignet sind. In der Flüssigphase wird beim Umsatz schwer verdampfbarer Ausgangsstoffe beziehungsweise hoch siedender (Pyro)Carbonate gearbeitet, wobei im Falle wenig reaktiver Ausgangsstoffe durch Druckerhöhung eine höhere Reaktionstemperatur zur Reaktionsbeschleunigung eingestellt werden kann. Bei leicht verdampfbaren Ausgangsstoffen wird in vielen Fällen die technisch günstige Gasphasen-Umsetzung gewählt.

Das erfindungsgemäße Verfahren kann im weiten Temperaturbereich von 80 bis 450°C durchgeführt werden. Zur Durchführung in der Gasphase wird bevorzugt im oberen Teil dieses Bereiches und zur Durchführung in der Flüssigphase bevorzugt im unteren Teil dieses Bereiches gearbeitet. Für die Gasphase sei daher bevorzugt ein Bereich von 250 bis 400°C, besonders bevorzugt von 300 bis 350°C genannt. Für die Flüssigphase, einschließlich der Rieselphase und gegebenenfalls unter erhöhtem Druck, sei daher bevorzugt ein Bereich von 80 bis 300°C, besonders bevorzugt ein Bereich von 80 bis 250°C, ganz besonders bevorzugt ein Bereich von 100 bis 200°C genannt.

So kann beispielsweise eine Mischung aus dem Lactam(IV), dem Carbonat oder Pyrocarbonat (V) sowie gegebenenfalls einem Lösungsmittel und gegebenenfalls einem Katalysator in einen Reaktor gebracht werden und dort unter Normaldruck oder unter erhöhtem Druck (in einem Autoklaven) auf Reaktionstemperatur gebracht werden und bei dieser Temperatur umgesetzt werden. Beim Arbeiten in einem Autoklaven kann hierbei bevorzugt unter dem sich automatisch einstellenden Druck (CO₂) gearbeitet werden.

Bei einer weiteren Form der Verfahrensdurchführung leitet man das Lactam(IV) und das Carbonat oder Pyrocarbonat (V), wobei die Dosierung als Gemisch oder in getrennten Stoffströmen erfolgt, durch einen Durchflußreaktor, in welchem inerte Füllkörper oder Füllkörper, bestehend aus einem oder mehreren der genannten Katalysatoren mit sauren und/oder basischen Zentren (gegebenenfalls Gemische aus inerten Füllkörpern und Katalysatoren) angeordnet sind. Diese Anordnung im Durchflußreaktor kann als Festbett, als Fließbett oder als Wirbelschicht vorhanden sein. Zu dieser Verfahrensführung werden die umzusetzenden Stoffe (IV) und (V) in die Gasphase übergeführt. Diese Überführung in die Gasphase kann auch vor dem Reaktor in einem Vorverdampfer vorgenommen werden. Bei dieser Form der Verfahrensdurchführung können die umzusetzenden Reaktionspartner (IV) und (V) auch getrennt oder gemeinsam als Lösung in einem der genannten Lösungsmittel eingesetzt werden; der entstehende Dampf des Lösungsmittels dient dann in der Flüssigphase zum besseren Dosieren und in der Gasphase als Träger.

In einer noch weiteren Variante der Verfahrensdurchführung werden die in die Gasform übergeführten umzusetzenden Stoffe (IV) und (V), gegebenenfalls zusammen mit einem Lösungsmittel, über den in einem Festbett befindlichen Katalysator geleitet, der zusätzlich mit einer stationären Flüssigphase, z.B. Polyethylenglykol, beschichtet ist (Gas-Flüssig- Phasentranaferkatalyse).

In einer noch weiteren Form der Verfahrensdurchführung können inerte Füllkörper, Katalysatoren mit sauren und/oder basischen Zentren oder Gemischen aus inerten Füllkörpern und Katalysatoren mit aktiven Zentren als Festbett in einem Durchflußreaktor angebracht sein und das umzusetzende Gemisch aus (IV) und (V) in der Flüssigphase darüber geleitet werden (Rieselphasen-Verfahren). Auch bei dieser Form der Verfahrensdurchführung kann in zusätzlicher Weise ein Lösungsmittel eingesetzt werden.

Für den Fall, daß in einem Durchflußreaktor in der Gasphase oder in der Rieselphase gearbeitet wird, werden Katalysatorbelastungen (ausgedrückt in Gramm Ausgangsstoffe (IV) und (V) pro Gramm Katalysator pro Stunde) von 0,1-5 g/g/h, bevorzugt 0,25-2 g/g/h, besonders bevorzugt 0,5-1 g/g/h eingestellt.

### Beispiele

### Verzeichnis der in den Beispielen eingesetzten Katalysatoren:

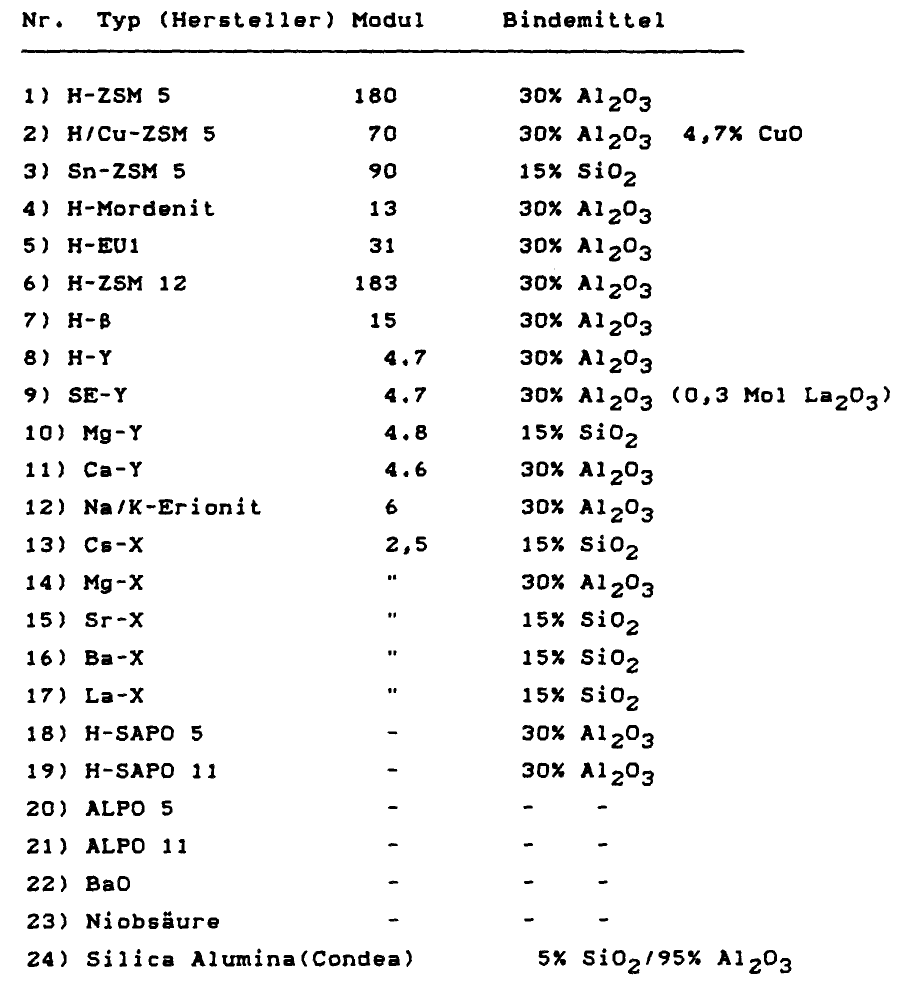

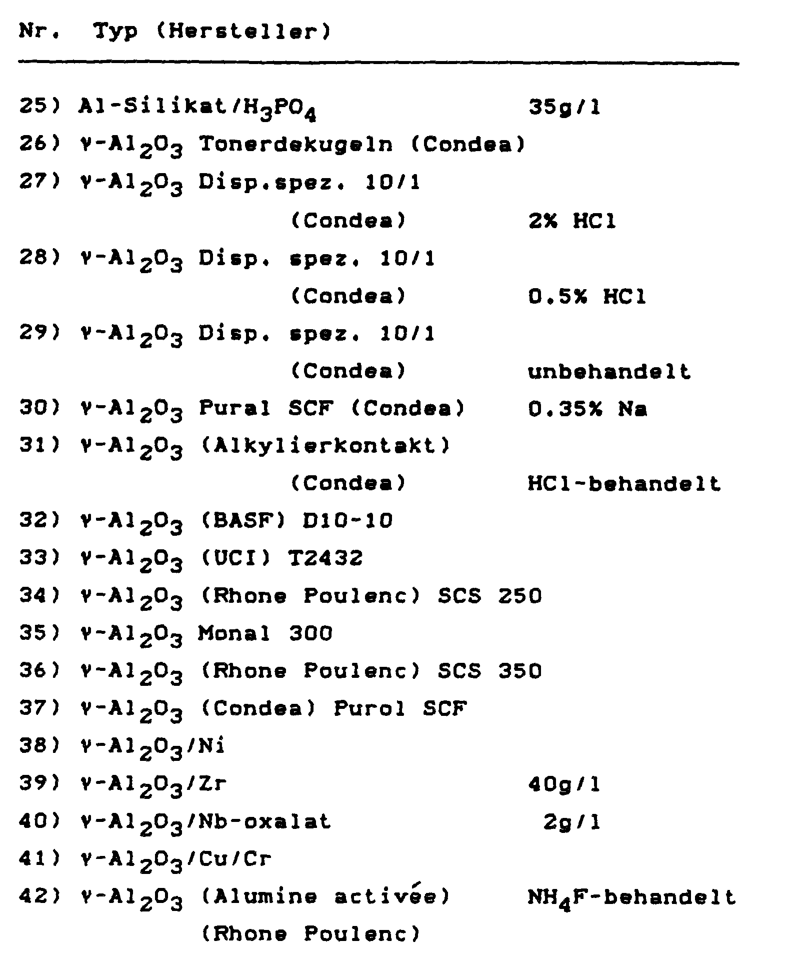

### Beispiele 1-32

4 Glasrohre von 250 mm Länge und 12 mm ø wurden mit Glaakügelchen von 3 mm ø und - in der Mitte der Rohre -6 ml Katalysator als Granulat befüllt und in einem Gaschrommtographieofen - senkrecht angeordnet - beheizt. Unter Durchleitung von Stickstoff (0.6 l/h) wurde 3 h bei 400°C calciniert, dann eine Lösung von 1 Mol Caprolactam in 4 Mol Dimethylcarbonat mit einer Förderungsrate von 3 ml/h in die Verdampferzone von 350°C eindosiert. Die Reaktionatemperatur betrug 300°C, die Menge des Trägergases 0.6 l N₂/h. Das am unteren Ende der Rohre austretende Reaktionsgemisch wurde in Kühlfallen kondensiert und der gaschromatografischen Bestimmung zugeführt.

Die Ergebnisse für Beispiele 1-32 sind in der Tabelle 1 zusammengestellt.

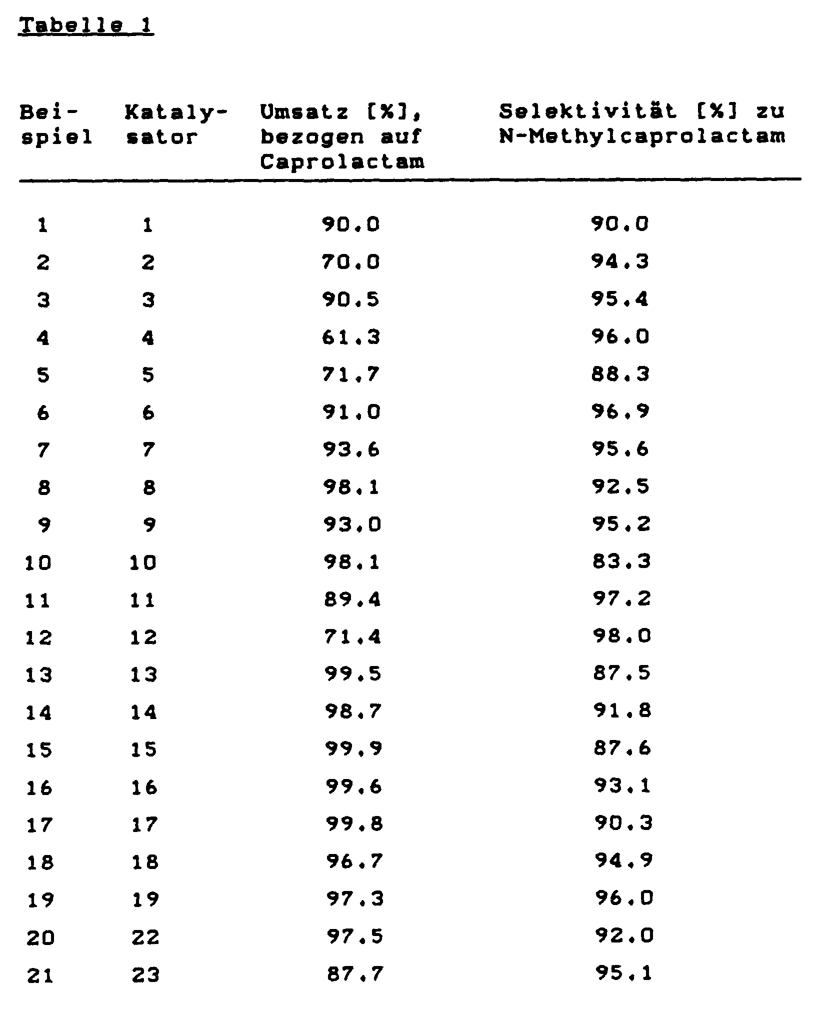

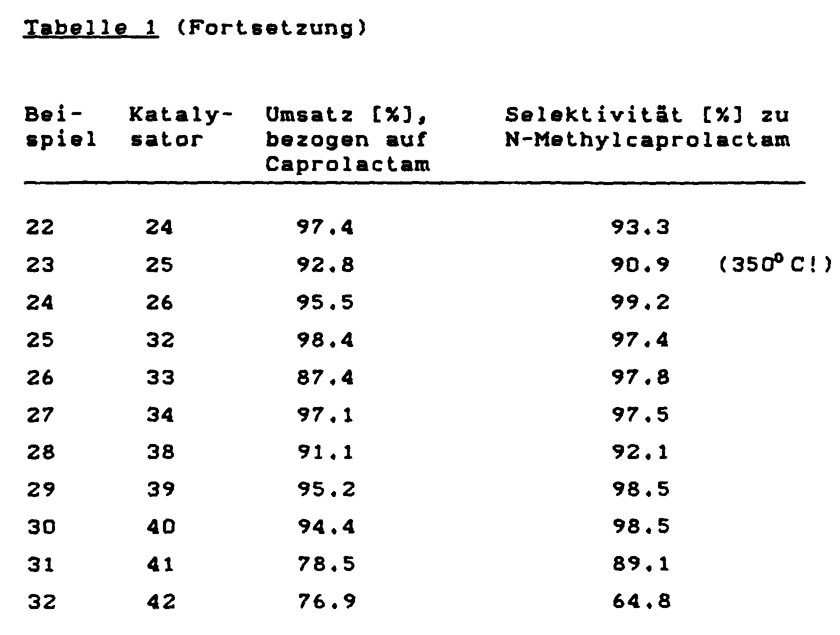

### Beispiel 33 - 36

In einer Versuchsanordnung wie in Beispielen 1-19 wurden bei gleicher Katalysator- und Produktstrommenge an Caprolactam und DMC der Katalysatortyp und die Reaktionstemperaturen variiert.

Die Befunde der gaschromatographischen Untersuchungen sind aus Tabelle 2 ersichtlich.

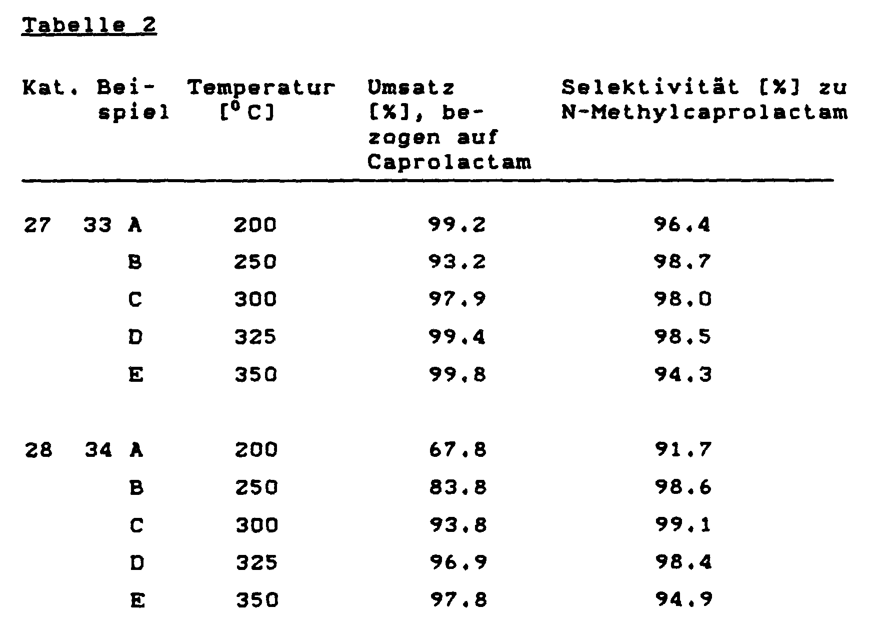

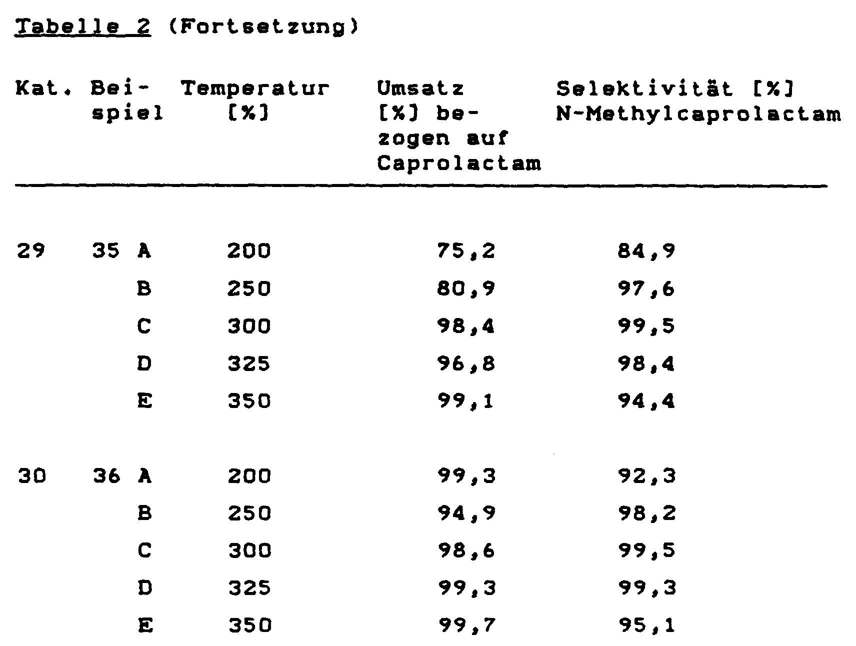

### Beispiele 37 - 41

In der gleichen Versuchsanordnung wie in den Beispielen 1-32 wurden beim Einsatz von jeweils 6 ml des Katalysators 27 3 ml/h einer Lösung von Caprolactam und Dimethylcarbonat in einem Lösungsmittel am Katalysator zur Reaktion gebracht. Über die Ergebnisse bei Verwendung unterschiedlicher Mischungsverhältnisse an Caprolactam/Dimethylcarbonat/Lösungsmittel und Reaktionstemperaturen gibt Tabelle 3 Auskunft.

**Tabelle 3**

| Beispiel | Reakt. temp. [°C] | Lösungsmittel [Gew.-%] | Molverhältnis Caprolactam/DMC | Umsatz, bez. auf Caprolactam | Selektivität zu NMC |
|---|---|---|---|---|---|
| 37 A | 300 | Benzol (60) | 1/1.2 | 89.5 | 99.7 |
| B | 350 | Benzol (60) | 1/1.2 | 99.5 | 97.6 |
| 38 A | 300 | Benzol (60) | 1/1.5 | 92.0 | 99.1 |
| B | 350 | Benzol (60) | 1/1.5 | 99.4 | 97.1 |
| 39 A | 300 | Methanol (33,3) | 1/1 | 62.2 | 98.7 |
| B | 350 | Methanol (33,3) | 1/1 | 92.5 | 99.3 |
| 40 A | 300 | Methanol (20) | 1/1.5 | 67.2 | 98.2 |
| B | 350 | Methanol (20) | 1/1.5 | 96.2 | 97.1 |
| 41 A | 300 | NMC (33,3) | 1/1.2 | 65.0 | 99.8 |
| B | 350 | NMC (33,3) | 1/1.2 | 92.0 | 99.6 |

### Beispiel 42

In einem ölbeheizten Glasrohrreaktor von 30 cm Länge und 5 cm ø wurden 30 g an Katalysator 31, eingebettet in der Mitte einer Füllung von Glaskugeln mit 5 mm ø, in einem Stickstoffstrom von 6 l/h 3 h bei 340°C calciniert. Anschließend dosierte man in die Verdampferzone oberhalb des Kontaktes gleichzeitig 7.55 g (0.067 Mol)/h Caprolactam aus einer bei 90°C gehaltenen Schmelze sowie 9,0 g (0.1 Mol)/h Dimethylcarbonat ein und leitete nach Verdampfung das Gemisch zusammen mit 6 l/h N₂ als Trägergas über den auf 325°C gehaltenen Kontakt. Das in einer Kühlfalle als Kondensat aufgefangene Reaktionsgemisch wurde der gaschromatographischen Untersuchung zugeführt. Nach 1 h betrug der Umsatz, bezogen auf Caprolactam, 95%, die Selektivität an N-Methylcaprolactam 97%.

### Beispiel 43 - 50

Bei gleicher Verfahrensweise wie in Beispiel 42 wurden 15 g/h einer bei 40°C in Lösung gehaltenen Mischung aus Caprolactam und Dimethylcarbonat im Molverhältnis 1:1,5 in die Verdampfungszone eindosiert und mit N₂ als Trägergas (6 l/h) bei Reaktionstemperatur über den Katalysator (30 g) geleitet. Umsätze, bezogen auf Caprolactam, sowie die Selektivität an N-Methylcaprolactam gehen aus Tabelle 4 hervor :

**Tabelle 4**

| Beispiel | Katalysator | Temp. [°C] | Umsatz [%] | Selektivität [%] |
|---|---|---|---|---|
| 43 | 31 | 325 | 95.7 | 98.7 |
| 44 | 18 | 345 | 90.5 | 98.3 |
| 45 | 19 | 345 | 93.5 | 97.8 |
| 46 | 20 | 345 | 98.2 | 93.6 |
| 47 | 21 | 345 | 98.5 | 93.4 |
| 48 | 35 | 345 | 98.8 | 98.2 |
| 49 | 36 | 345 | 97.5 | 95.1 |
| 50 | 37 | 345 | 99.4 | 95.2 |

### Beispiel 51 - 54

In einer Versuchsführung analog Beispielen 1-32 wurde Lactam-N-Carbonwäuremethylester in 33.3%iger Lösung in Benzol mit einer Fördergeschwindigkeit von 3 ml/h in die 350°C heiße Verdampfungszone eindosiert und mit 0.6 l/h N₂ als Trägergas über 6 ml verschiedene Katalysatoren geleitet. Die Reaktionstemperatur betrug 350°C. Die Ergebnisse der gaschromatographischen Bestimmung sind in Tabelle 5 zusammengestellt.

**Tabelle 5**

| Beispiel | Katalysator | Umsatz [%] Lactamcarbonsäureester | Selektivität [%] zu N-Methylcaprolactam |
|---|---|---|---|
| 51 | 1 | 100 | 72.3 |
| 52 | 9 | 100 | 70.5 |
| 53 | 19 | 100 | 81.9 |
| 54 | 27 | 100 | 86.8 |

### Beispiele 55 - 58

Analog den Beispielen 1-32 wurde eine Mischung aus Pyrrolidon und Dimethylcarbonat im Molverhältnis 1:1.5 mit einer Fördergeschwindigkeit von 3 ml/h an 6 ml-Proben verschiedener Katalysatoren umgesetzt. Reaktionstemperaturen, Katalysatoren und Ausbeuteergebnisse sind in Tabelle 6 verzeichnet.

**Tabelle 6**

| Beispiel | Katalysator | Reakt. temp. [°C] | Umsatz [%], bezogen auf Pyrrolidon | Selektivität [%] zu N-Methylpyrrolidon |
|---|---|---|---|---|
| 55 A | 27 | 300 | 99.1 | 99.1 |
| B | 27 | 350 | 99.8 | 98.6 |
| 56 A | 28 | 300 | 94.5 | 98.9 |
| B | 28 | 350 | 99.9 | 98.4 |
| 57 A | 29 | 300 | 92.0 | 99.0 |
| B | 29 | 350 | 99.9 | 98.3 |
| 58 A | 30 | 300 | 100 | 98.0 |
| B | 30 | 350 | 99.9 | 98.0 |

### Beispiele 59 - 64

Analog den Beispielen 1-32 wurde ein Gemisch aus Caprolactam und Diethylcarbonat im Molverhältnis 1:4 eingesetzt. Reaktionsdaten und Ausbeuteangaben sind aus Tabelle 7 ersichtlich.

**Tabelle 7**

| Beispiel | Katalysator | Reakt. temp. [°C] | Umsatz [%], bezogen auf Caprolcatam | Selektivität [%] zu N-Ethylcaprolactam |
|---|---|---|---|---|
| 59 A | 27 | 300 | 77.2 | 98.6 |
| B | | 350 | 98.3 | 93.1 |
| 60 A | 28 | 300 | 68.9 | 98.6 |
| B | | 350 | 96.1 | 92.2 |
| 61 A | 29 | 300 | 65.9 | 98.4 |
| B | | 350 | 97.8 | 90.0 |
| 62 A | 30 | 300 | 86.3 | 98.6 |
| B | | 350 | 98.7 | 88.8 |
| 63 | 6 | 300 | 65.1 | 78.3 |
| 64 | 7 | 300 | 68.5 | 93.9 |

### Beispiele 65 - 68

Analog den Beispielen 1-32 setzte man eine 66.6Xige Lösung von Caprolactam und Dipropylcarbonat in Benzol an verschiedenen Katalysatoren um. Reaktionsdaten und Ausbeuteangaben sind in Tabelle 8 zusammengestellt.

**Tabelle 8**

| Beispiel | Katalysator | Reakt. temp. [°C] | Umsatz [%], bezogen auf Caprolcatam | Selektivität [%] zu N-Propylcaprolactam |
|---|---|---|---|---|
| 65 A | 27 | 300 | 49.5 | 93.3 |
| B | 27 | 350 | 98.0 | 96.1 |
| 66 A | 28 | 300 | 49.8 | 94.5 |
| B | 28 | 350 | 87.4 | 96.2 |
| 67 A | 29 | 300 | 48.2 | 95.1 |
| B | 29 | 350 | 88.5 | 96.4 |
| 68 A | 30 | 300 | 58.7 | 95.7 |
| B | 30 | 350 | 94.1 | 95.4 |

### Beispiel 69

In einem 500 ml-Autoklaven wurde eine Mischung aus 28,5 g (0,3 Mol) Pyridon-2, 81,1 g (O,9 Mol) Dimethylcarbonat und 6 g K₂CO₃ unter 10 bar N₂-Anfangsdruck auf 220°C aufgeheizt und 6 h bei dieser Temperatur gehalten. Nach Abkühlen wurde in Toluol aufgenommen, vom Katalysator abgesaugt, die organische Phase eingeengt und destilliert: Ausbeute 29 g (88,6 %) helles Öl vom Kp 254- 256°C; nach GC betrug die Reinheit 99.9 % an N-Methylpyridon-2.

### Beispiel 70

Eine Mischung aus 14,3 g (0,15 Mol) Pyridon-2, 40,5 g (0,45 Mol) DMC und 2 g K₂CO₃ wurde 24 h am Rückfluß erhitzt (94 → 83°C). Die GC-Untersuchung des Reaktionsgemisches ergab 100 % Umsatz und 99,0 % Selektivität an N-Methylpyridon-2.

### Beispiel 71

33,8 g (0,2 Mol) Naphtholactam, 70,9 g (0,6 Mol) Diethylcarbonat und 5 g K₂CO₃-Pulver wurden in einem 250 ml Autoklav mit N₂ auf einen Anfangswert von 10 bar eingestellt, auf 250°C aufgeheizt und 6 h bei dieser Temperatur gehalten (84 bar), Lt. GC betrug der Umsatz 97,9 %, die Selektivität an N-Ethyl-naphtholactam 99 %.

### Beispiel 72

Bei gleicher Verfahrenweise wie in Beispiel 71 betrug nach 6 (12) h und 200°C der Umsatz 71,7 (97,4)% und die Selektivität an N-Ethyl-naphtholactam 99 %.

### Beispiel 73

Setzte man die Ausgangsprodukte gemäß Beispiel 71 bei Normaldruck unter Rückflußbedingungen (125°C) um, so erhielt man nach 40 h bei einem Umsatz von 80 % eine 99 %ige Selektivität an N-Ethyl-naphtholactam.

### Beispiel 74

Unter den gleichen Bedingungen wie in Beispiel 71 wurden 1 g (10⁻³ Mol) Phenanthridinon, 9 g (0,1 Mol) Dimethylcarbonat sowie 0,5 g K₂CO₃-Pulver miteinander umgesetzt. Nach 6 h bei 220°C betrug der Umsatz 99,1 % und die Selektivität an N-Methylphenanthridinon 87 %.

## Patentansprüche

1. Verfahren zur Herstellung von N-substituierten Lactamen der Formel 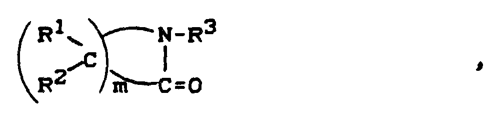 in der
m eine ganze Zahl von 2 bis 12 bedeutet,
R¹ und R² unabhängig voneinander Wasserstoff, geradkettiges oder verzweigtes C₁-C₆-Alkyl, geradkettiges oder verzweigtes C₃-C₆-Alkenyl, C₅-C₇-Cycloalkyl, Benzyl, Phenyl, Fluor, Chlor oder Brom darstellen, wobei an der durch den Index m bezeichneten Zahl von C-Ringgliedern höchstens 4 von Wasserstoff verschiedene Substituenten R¹ und R² vorhanden sind und wobei weiterhin eines oder zwei der in Klammern gesetzten C-Ringglieder durch -N(CH₃)- oder -N(C₂H₅)- ersetzt sein können, mehrere dieser C-Ringglieder durch Doppelbindungen verknüpft sein können oder zwei oder mehrere der in Klammern gesetzen C-Ringglieder Teile eines weiteren, aromatischen oder cycloaliphatischen Ringes sein können und die Reste R¹ und R² die ergänzenden Teile eines solchen Ringes darstellen, und
R³ geradkettiges oder verzweigtes C₁-C₈-Alkyl, C₃-C₈-Alkenyl oder C₃-C₈-Alkinyl oder C₅-C₇-Cycloalkyl bedeutet,
dadurch gekennzeichnet, daß man Lactam-N-carbonsäureester oder Lactim-O-carbonsäureester der Formeln 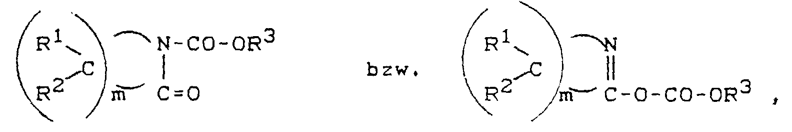 in denen m, R¹, R² und R³ die obige Bedeutung haben,
bei Temperaturen von 80-450°C in der Gas- oder der Flüssigphase und in Gegenwart oder Abwesenheit von Katalysatoren mit sauren und/oder basischen Zentren und in Gegenwart oder Abwesenheit eines inerten Lösungsmittels unter CO₂-Austritt spaltet, wobei im Falle des Einsatzes von Lactim-O-carbonsäureestern die intermediär entstehenden Lactim-ether unter den Reaktionsbedingungen in die N-substituierten Lactame umgelagert werden.

2. Verfahren nach Anspruch 1, dadurch gekennzeichnet, daß die einzusetzenden Lactam-N-carbonsäureester und Lactim-O-carbonsäureester durch Umsetzung von Lactamen der Formel 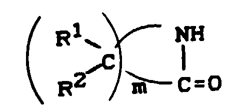 mit Carbonaten oder Pyrocarbonaten der Formel
R³O-CO-(O-CO)ₙ-OR³
worin
m eine ganze Zahl von 2 bis 12 und
n 0 oder 1 bedeuten,
R¹ und R² unabhängig voneinander Wasserstoff, geradkettiges oder verzweigtes C₁-C₆-Alkyl, geradkettiges oder verzweigtes C₃-C₆-Alkenyl, C₅-C₇-Cycloalkyl, Benzyl, Phenyl, Fluor, Chlor oder Brom darstellen, wobei an der durch den Index m bezeichneten Zahl von C-Ringgliedern höchstens 4 von Wasserstoff verschiedene Substituenten R¹ und R² vorhanden sind und wobei weiterhin eines oder zwei der in Klammern gesetzten C-Ringglieder durch -N(CH₃)- oder -N(C₂H₅)- ersetzt sein können, mehrere dieser C-Ringglieder durch Doppelbindungen verknüpft sein können oder zwei oder mehrere der in Klammern gesetzten C-Ringglieder Teile eines weiteren, aromatischen oder cycloaliphatischen Ringes sein können und die Reste R¹ und R² die ergänzenden Teile eines solchen Ringes darstellen, und
R³ geradkettiges oder verzweigtes C₁-C₈-Alkyl, C₃-C₈-Alkenyl oder C₃-C₆-Alkinyl oder C₅-C₇-Cycloalkyl bedeutet,
bei Temperaturen von 80-450°C in der Gas- oder der Flüssigphase und in Gegenwart oder Abwesenheit von Katalysatoren mit sauren und/oder basischen Zentren und in Gegenwart oder Abwesenheit eines inerten Lösungsmittels in situ hergestellt werden und ohne Zwischenisolierung unter den gleichen Bedingungen und unter CO₂-Austritt in die N-substituierten Lactame umgewandelt werden.

3. Verfahren nach Anspruch 1, dadurch gekennzeichnet, daß m die Zahl 3, 4, 5 oder 12 bedeutet.

4. Verfahren nach Anspruch 1, dadurch gekennzeichnet, daß R¹ und R² durch R¹¹ und R¹² ersetzt werden, wobei
R¹¹ Wasserstoff, geradkettiges oder verzweigtes C₁-C₄-Alkyl, Allyl, Butenyl, C₅-C₇-Cycloalkyl, Benzyl, Phenyl oder Chlor bedeutet und
R¹² für Wasserstoff, geradkettiges oder verzweigtes C₁-C₄-Alkyl oder Chlor steht.

5. Verfahren nach Anspruch 4, dadurch gekennzeichnet, daß R¹¹ und R¹² durch R²¹ und R²² ersetzt werden, wobei
R²¹ Wasserstoff, Methyl, Ethyl, Benzyl oder Phenyl bedeutet und
R²² für Wasserstoff, Methyl oder Ethyl steht.

6. Verfahren nach Anspruch 1 und 2, dadurch gekennzeichnet, daß der Formelteil 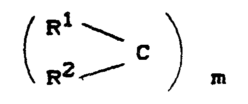 eine Kohlenstoffkette mit 2 oder 3 konjugierten C-C-Doppelbindungen, den 1,8-Naphthylen-Rest, den 2,2'-Biphenylen-Rest, den o-Phenylen-ethylen-Rest oder den o-Phenylen-vinylen-Rest darstellt.

7. Verfahren nach Anspruch 1, dadurch gekennzeichnet, daS R³ durch R¹³ mit der Bedeutung geradkettiges oder verzweigtes C₁-C₄-Alkyl, Allyl oder Propargyl ersetzt wird, bevorzugt dadurch gekennzeichnet, daß R¹³ durch R²³ mit der Bedeutung Methyl oder Ethyl ersetzt wird.

8. Verfahren nach Anspruch 1, dadurch gekennzeichnet, daß in Gegenwart eines Katalysators mit sauren und/oder basischen Zentren aus der Gruppe der Tonmineralien, Säuren, freie Säuren oder Säuregruppen enthaltenden Träger, sauer reagierenden Metalloxide und Metallsulfide, Metallsalze, gemischten Oxide, festen Supersäuren, der sauren und der mit Metallkationen ausgetauschten Zeolithe, der Alkali- und Erdalkalimetallhydroxide, -oxide, -alkoholate, -hydride, hydrogencarbonate und -carbonate gearbeitet wird.

9. Verfahren nach Anspruch 1, dadurch gekennzeichnet, daß in der Gasphase in einem Temperaturbereich von 250 bis 400°C, bevorzugt 300 bis 350°C gearbeitet wird.

10. Verfahren nach Anspruch 1, dadurch gekennzeichnet, daß in der Flüssigphase in einem Temperaturbereich von 80 bis 300°C, bevorzugt von 80 bis 250°C, besonders bevorzugt 100 bis 200°C gearbeitet wird.

## Claims

1. Process for the preparation of N-substituted lactams of the formula 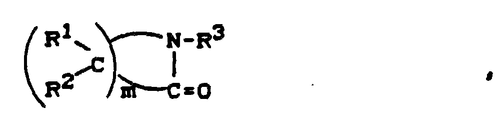 in which
m denotes an integer from 2 to 12,
R¹ and R² independently of one another represent hydrogen, straight-chain or branched C₁-C₆-alkyl, straight-chain or branched C₃-C₆-alkenyl, C₅-C₇-cycloalkyl, benzyl, phenyl, fluorine, chlorine or bromine, at most four substituents R¹ and R² other than hydrogen being present on the number of C ring members designated by the index m and it furthermore being possible to replace one or two of the C ring members in brackets by -N(CH₃) - or -N(C₂H₅) -, it being possible to link several of these C ring members by double bonds or it being possible for two or more of the C ring members in brackets to be parts of a further aromatic or cycloaliphatic ring, and the radicals R¹ and R² representing the supplementary parts of such a ring, and
R³ denotes straight-chain or branched C₁-C₈-alkyl, C₃-C₈-alkenyl or C₃-C₈-alkinyl or C₅-C₇-cycloalkyl,
which is characterised in that lactam N-carboxylates or lactim O-carboxylates of the formulae 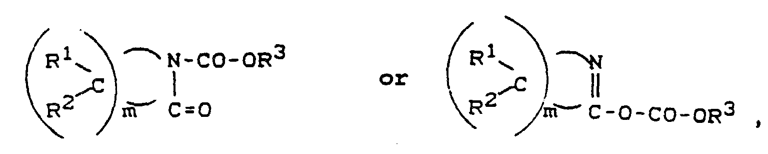 in which m, R¹, R² and R³ have the above meaning,
are cleaved, with elimination of CO₂, at temperatures of 80 - 450°C in the gas or the liquid phase and in the presence or absence of catalysts having acidic and/or basic centres and in the presence or absence of an inert solvent, in the case of the use of lactim O-carboxylates the intermediately formed lactim ethers being rearranged under the reaction conditions to the N-substituted lactams.

2. Process according to Claim 1, characterised in that the lactam N-carboxylates and lactim O-carboxylates to be employed are prepared in situ by reaction of lactams of the formula 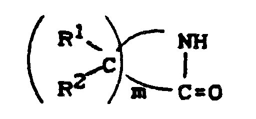 with carbonates or pyrocarbonates of the formula
R³O-CO-(O-CO)ₙ-OR³
in which
m denotes an integer from 2 to 12 and
n denotes 0 or 1,
R¹ and R² independently of one another represent hydrogen, straight-chain or branched C₁-C₆-alkyl, straight-chain or branched C₃-C₆-alkenyl, C₅-C₇-cycloalkyl, benzyl, phenyl, fluorine, chlorine or bromine, at most four substituents R¹ and R² other than hydrogen being present on the number of C ring members designated by the index m and it furthermore being possible to replace one or two of the C ring members in brackets by -N(CH₃) - or -N(C₂H₅)-, it being possible to link several of these C ring members by double bonds or it being possible for two or more of the C ring members in brackets to be parts of a further aromatic or cycloaliphatic ring, and the radicals R¹ and R² representing the supplementary parts of such a ring, and
R³ denotes straight-chain or branched C₁-C₈-alkyl, C₃-C₈-alkenyl or C₃-C₈-alkinyl or C₅-C₇-cycloalkyl,
at temperatures of 80 - 450°C in the gas or the liquid phase and in the presence or absence of catalysts having acidic and/or basic centres and in the presence or absence of an insert solvent, and are converted into the N-substituted lactams under the same conditions and with CO₂ elimination without intermediate isolation.

3. Process according to Claim 1, characterised in that m denotes the number 3, 4, 5 or 12.

4. Process according to Claim 1, characterised in that R¹ and R² are replaced by R¹¹ and R¹²,
where
R¹¹ denotes hydrogen, straight-chain or branched C₁-C₄-alkyl, allyl, butenyl, C₅-C₇-cycloalkyl, benzyl, phenyl or chlorine and
R¹² represents hydrogen, straight-chain or branched C₁-C₄-alkyl or chlorine.

5. Process according to Claim 4, characterised in that R¹¹ and R¹² are replaced by R²¹ and R²²,
where
R²¹ denotes hydrogen, methyl, ethyl, benzyl or phenyl and
R²² represents hydrogen, methyl or ethyl.

6. Process according to Claim 1 and 2, characterised in that the formula moiety 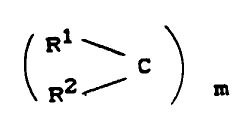 represents a hydrocarbon chain having 2 or 3 conjugated C-C double bonds, the 1,8-naphthylene radical, the 2,2'-biphenylene radical, the o-phenyleneethylene radical or the o-phenylene-vinylene radical.

7. Process according to Claim 1, characterised in that R³ is replaced by R¹³ having the meaning straight-chain or branched C₁-C₄-alkyl, allyl or propargyl, preferably characterised in that R¹³ is replaced by R²³ having the meaning methyl or ethyl.

8. Process according to Claim 1, characterised in that it is carried out in the presence of a catalyst having acidic and/or basic centres from the group comprising the clay minerals, carriers containing acids, free acids or acid groups, acid-reacting metal oxides and metal sulphides, metal salts, mixed oxides, solid super-acids, acidic zeolites and zeolites exchanged with metal cations, alkali metal and alkaline earth metal hydroxides, oxides, alkoxides, hydrides, hydrogencarbonates and carbonates.

9. Process according to Claim 1, characterised in that it is carried out in the gas phase in a temperature range from 250 to 400°C, preferably 300 to 350°C.

10. Process according to Claim 1, characterised in that it is carried out in the liquid phase in a temperature range from 80 to 300°C, preferably from 80 to 200°C, particularly preferably 100 to 150°C.

## Revendications

1. Procédé pour la préparation de lactames substitués à l'azote répondant à la formule 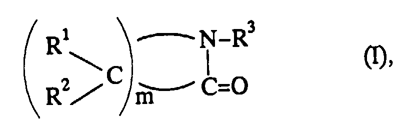 dans laquelle
m est un nombre entier allant de 2 à 12,
R¹ et R² représentent chacun, indépendamment l'un de l'autre, l'hydrogène, un groupe alkyle à chaîne droite ou ramifiée en C₁-C₆, un groupe alcényle à chaîne droite ou ramifiée en C₃-C₆, un groupe cycloalkyle en C₅-C₇, benzyle, phényle, le fluor, le chlore ou le brome, sous réserve que, sur les chaînons cycliques carbonés dont le nombre est indiqué par l'indice m, il y a au maximum 4 substituants R¹ et R² autres que l'hydrogène, que d'autre part 1 ou 2 des chaînons cycliques carbonés figurant entre parenthèses peuvent être remplacés par -N(CH₃)- ou -N(C₂H₅)-, plusieurs de ces chaînons cycliques carbonés pouvant être reliés entre eux par des doubles liaisons, ou bien deux ou plusieurs des chaînons cycliques carbonés représentés entre parenthèses peuvent faire partie d'un autre noyau aromatique ou cycloaliphatique complété par les groupes R¹ et R², et
R³ représente un groupe alkyle en C₁-C₈, alcényle en C₃-C₈ ou alcynyle en
C₃-C₈ à chaîne droite ou ramifiée ou un groupe cycloalkyle en C₅-C₇, caractérisé en ce que l'on scinde des esters d'acides lactame-N-carboxyliques ou des esters d'acides lactame-O-carboxyliques de formules respectives 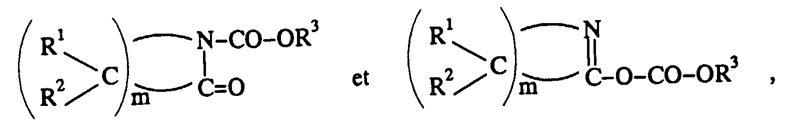 dans lesquelles m, R¹, R² et R³ ont les significations indiquées ci-dessus, à des températures de 80 à 450°C, en phase gazeuse ou liquide et en présence ou en l'absence de catalyseurs à centres acides et/ou basiques et en présence ou en l'absence d'un solvant inerte, avec séparation de CO₂, et, dans le cas où on a mis en oeuvre des esters d'acides lactime-O-carboxyliques, on transpose les éthers de lactimes formés en produits intermédiaires, dans les conditions de la réaction, en les lactames substitués à l'azote.

2. Procédé selon la revendication 1, caractérisé en ce que les esters d'acides lactame-N-carboxyliques et esters d'acides lactime-O-carboxyliques à mettre en oeuvre sont eux-mêmes préparés in situ par réaction de lactames de formule 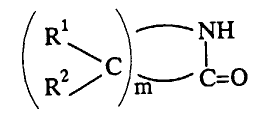 avec des carbonates ou pyrocarbonates de formule
R³O-CO-(O-CO)ₙ-OR³
dans lesquelles
m est un nombre entier allant de 2 à 12 et
n est égal à 0 ou 1,
R¹ et R² représentent chacun, indépendamment l'un de l'autre, l'hydrogène, un groupe alkyle à chaîne droite ou ramifiée en C₁-C₆, un groupe alcényle à chaîne droite ou ramifiée en C₃-C₆, un groupe cycloalkyle en C₅-C₇, benzyle, phényle, le fluor, le chlore ou le brome, sous réserve que, sur les chaînons cycliques carbonés dont le nombre est indiqué par l'indice m, il y a au maximum 4 substituants R¹ et R² autres que l'hydrogène et que d'autre part 1 ou 2 des chânons cycliques carbonés figurant entre parenthèses peuvent être remplacés par -N(CH₃)- ou -N(C₂H₅)-, plusieurs de ces chaînons carbonés peuvent être reliés par des doubles liaisons, ou bien deux ou plusieurs des chaînons carbonés figurant entre parenthèses peuvent faire partie d'un autre noyau aromatique ou cycloaliphatique qui est complété par les groupes R¹ et R², et
R³ représente un groupe alkyle en C₁-C₈, alcényle en C₃-C₈ ou alcynyle en C₃-C₈ à chaîne droite ou ramifiée ou un groupe cycloalkyle en C₅-C₇,
à des températures de 80 à 450°C en phase gazeuse ou liquide et en présence ou en l'absence de catalyseurs à centres acides et/ou basiques et en présence ou en l'absence d'un solvant inerte, et convertis, sans être isolés, dans les mêmes conditions, avec séparation de CO₂, en les lactames substitués à l'azote.

3. Procédé selon la revendication 1, caractérisé en ce que m est égal à 3, 4, 5 ou 12.

4. Procédé selon la revendication 1, caractérisé en ce que R¹ et R² sont remplacés par R¹¹ et R¹² qui ont les significations suivantes :
R¹¹ représente l'hydrogène, un groupe alkyle à chaîne droite ou ramifiée en C₁-C₄, un groupe allyle, butényle, cycloalkyle en C₅-C₇, benzyle, phényle ou le chlore et
R¹² représente l'hydrogène, un groupe alkyle à chaîne droite ou ramifiée en C₁-C₄ ou le chlore.

5. Procédé selon la revendication 4, caractérisé en ce que R¹¹ et R¹² sont remplacés par R²¹ et R²² ayant les significations suivantes :
R²¹ représente l'hydrogène, un groupe méthyle, éthyle, benzyle ou phényle et
R²² représente l'hydrogène, un groupe méthyle ou éthyle.

6. Procédé selon les revendications 1 et 2, caractérisé en ce que la partie de formule 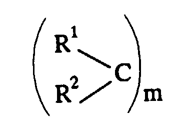 représente une chaîne carbonée à 2 ou 3 doubles liaisons conjuguées C-C, le groupe 1,8-naphtylène, le groupe 2,2'-biphénylène, le groupe o-phényléne-éthylène ou le groupe o-phénylène-vinylène.

7. Procédé selon la revendication 1, caractérisé en ce que R³ à R¹³ représentent des groupes alkyles à chaîne droite ou ramifiée en C₁-C₄, allyle ou propargyle, et de préférence R³ à R²³ représentent des groupes méthyle ou éthyle.

8. Procédé selon la revendication 1, caractérisé en ce que l'on opère en présence d'un catalyseur à centres acides et/ou basiques choisi parmi les minéraux argileux, les acides, les véhicules contenant des acides libres ou des groupes acides, les oxydes métalliques et sulfures métalliques, sels métalliques à réaction acide, oxydes mélangés, superacides solides, zéolithes acides et échangées contre des cations métalliques, hydroxyde, oxydes, alcoolates, hydrures, bicarbonates et carbonates de métaux alcalins et alcalino-terreux.

9. Procédé selon la revendication 1, caractérisé en ce que l'on opère en phase gazeuse dans l'intervalle de température de 250 à 400°C, de préférence de 300 à 350°C.

10. Procédé selon la revendication 1, caractérisé en ce que l'on opère en phase liquide dans l'intervalle de température de 80 à 300°C, de préférence de 80 à 250°C et plus spécialement de 100 à 200°C.
